# EUROPÄISCHE PATENTSCHRIFT

(11) **EP 0 821 006 B1**
(45) Veröffentlichungstag und Bekanntmachung des Hinweises auf die Patenterteilung: **21.04.2004**
(21) Anmeldenummer: 97112197.5
(22) Anmeldetag: 17.07.1997
(51) Int. Cl.: C07K 14/62, A61K 38/28

(54) **Insulinderivate mit erhöhter Zinkbindung**
Derivatives of insulin having enhanced zinc binding activity
Dérivés de l'insuline ayant une activité de liaison au zinc augmentée

(30) Priorität: 26.07.1996 DE 19630242
(43) Veröffentlichungstag der Anmeldung: 28.01.1998
(73) Patentinhaber: Aventis Pharma Deutschland GmbH, 65929 Frankfurt am Main (DE)
(72) Erfinder: Ertl, Johann, Dr., 65817 Bremthal (DE); Habermann, Paul, Dr., 65817 Eppstein (DE); Geisen, Karl, Dr., 60318 Frankfurt (DE); Seipke, Gerhard, Dr., 65719 Hofheim (DE)

(56) Entgegenhaltungen:
- EP-A- 0 544 466
- DE-A- 3 327 709
- US-A- 5 177 058
- MARKUSSEN J ET AL: "SOLUBLE, PROLONGED-ACTING INSULIN DERIVATIVES. III. DEGREE OF PROTRACTION, CRYSTALLIZABILITY AND CHEMICAL STABILITY OF INSULINS SUBSTITUTED IN POSITIONS A21, B13, B23, B27 AND B30" PROTEIN ENGINEERING, OXFORD UNIVERSITY PRESS, SURREY, GB, Bd. 2, Nr. 2, 1. Juli 1988 (1988-07-01), Seiten 157-166, XP000006171 ISSN: 0269-2139
- SMITH GD ET AL.: "Structural stability in the 4-zinc human insulin hexamer" PROC. NATL. ACAD. SCI. USA, Bd. 81, Nr. 22, 1984, Seiten 7093-7097, XP001021868

## Beschreibung

Die Pharmakokinetik von subkutan appliziertem Insulin ist von seinem Assoziationsverhalten abhängig. Insulin bildet in neutraler wäßriger Lösung Hexamere. Wenn das Insulin aus dem Gewebe in den Blutstrom und an den Wirkort gelangen will, muß das Insulin zunächst die Wände der Kapillaren passieren. Man nimmt an, daß dies nur für monomeres und für dimeres Insulin möglich ist - nicht oder nur wenig jedoch für hexamere Insuline oder höhermolekulare Assoziate (Brange et al., Diabetes Care: 13 (1990), Seiten 923-954; Kang et al. Diabetes Care: 14 (1991), Seiten 942-948). Die Dissoziation des Hexamers ist daher Voraussetzung für den raschen Übergang aus dem subkutanen Gewebe in den Blutstrom.

Das Assoziations- und Aggregationsverhalten von Insulin wird durch Zink⁺⁺ beeinflußt, welches zu einer Stabilisierung des Hexamers und bei pH-Werten um den Neutralpunkt zur Ausbildung von höhermolekularen Aggregaten bis hin zur Präzipitation führt. Zink⁺⁺ als Zusatz zu einer ungepufferten Humaninsufin-lösung (pH 4) beeinflußt das Wirkprofil jedoch nur wenig. Obwohl solch eine Lösung nach der Injektion im subkutanen Gewebe rasch neutralisiert wird und sich Insulin-Zink-Komplexe bilden, reicht die natürliche Zinkbindung des Humaninsulins nicht aus, Hexamere und höhere Aggregate zu stabilisieren. Daher wird durch die Zugabe von Zink⁺⁺ die Freisetzung des Humaninsulins nicht wesentlich verzögert, und ein starker Depoteffekt wird nicht erzielt. Bekannte Insulinhexamere zeigen einen Gehalt von etwa 2 Mol Zink⁺⁺ pro Mol Insulinhexamer (Blundell et al., Adv. Protein Chem.: 26 (1972), Seiten 323-328). Zwei Zinkionen pro Insulinhexamer sind so fest mit dem Insulinhexamer verbunden und können nicht durch übliche Dialyse entfernt werden. Es sind zwar sogenannte 4-Zink-Insulinkristalle beschrieben worden, jedoch enthalten diese Kristalle im Mittel nur weniger als drei Mol Zink⁺⁺ pro Mol Insulinhexamer (G.D. Smith et al., Proc. Natl. Acad. Sci. USA: 81, Seiten 7093-7097).

Aufgabe der vorliegenden Erfindung ist es Insulinderivate zu finden, die ein erhöhtes Zinkbindungsvermögen aufweisen, einen stabilen Komplex enthaltend Insulinhexamer und Zink⁺⁺ bilden und ein verzögertes Wirkprofil bei der subkutanen Injektion im Vergleich mit Humaninsulin zeigen.

Es wurden nun Insuline der Formel I, 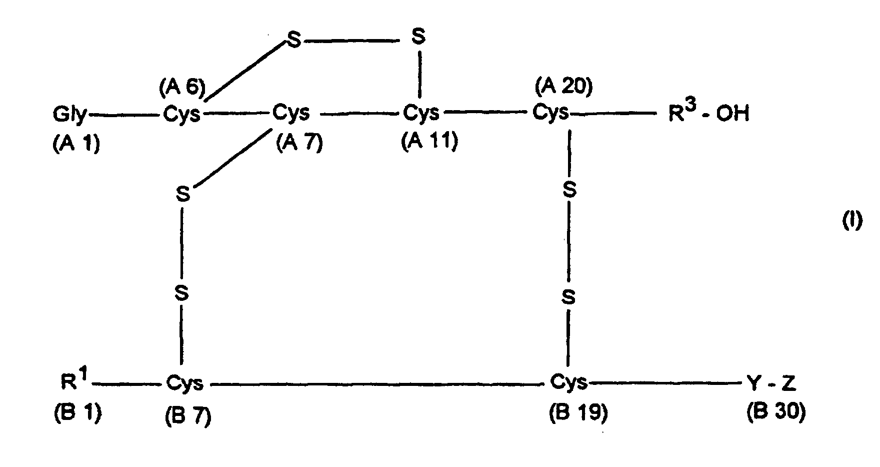 und/oder physiologisch verträgliche Salze der Insuline der Formel I gefunden, die die oben genannten Kriterien erfüllen und dadurch gekennzeichnet sind, daß
- R¹ für: einen Phenylalaninrest oder ein Wasserstoffatom steht,
- R³ für: einen genetisch kodierbaren Aminosäurerest steht,
- Y für: einen genetisch kodierbaren Aminosäurerest steht,
- Z für: a) den Aminosäurerest His oder
b) ein Peptid mit 2 bis 35 genetisch kodierbaren Aminosäureresten, enthaltend 1 bis 5 Histidinreste, steht,
und die Reste A2-A20 entsprechen der Aminosäuresequenz der A-Kette von Humaninsulin, tierischen Insulin oder einem Insulinderivat und die Reste B2-B29 entsprechen der Aminosäuresequenz der B-Kette von Humaninsulin, tierischem Insulin oder einem Insulinderivat.

Insbesondere bevorzugt ist ein Insulin der Formel I, wobei
- R¹ für: einen Phenylalaninrest steht,
- R³ für: einen Aminosäurerest aus der Gruppe Asn, Gly, Ser, Thr, Ala, Asp, Glu und Gln steht,
- Y für: einen Aminosäurerest aus der Gruppe Ala, Thr, Ser und His steht,
- Z für: a) den Aminosäurerest His oder
b) ein Peptid mit 4 bis 7 Aminosäureresten, enthaltend 1 oder 2 Histidinreste steht.

Ferner ist bevorzugt ein Insulin der Formel I, wobei
- R¹ für: einen Phenylalaninrest steht,
- R³ für: einen Aminosäurerest aus der Gruppe Asn, Gly, Ser, Thr, Ala, Asp, Glu und Gln steht,
- Y für: einen Aminosäurerest aus der Gruppe Ala, Thr, Ser und His steht,
- Z für: a) den Aminosäurerest His oder
b) ein Peptid mit 2 bis 7 Aminosäureresten, enthaltend 1 oder 2 Histidinreste steht.

Besonders bevorzugt ist ein Insulin der Formel I, wobei
- Z für: ein Peptid mit 1 bis 5 Aminosäureresten steht, enthaltend 1 oder 2 Histidinreste.

Insbesondere bevorzugt ist ein Insulin der Formel I, wobei
- R¹ für: einen Phenylalaninrest steht,
- R³ für: einen Aminosäurerest aus der Gruppe Asn und Gly steht,
- Y für: einen Aminosäurerest aus der Gruppe Thr und His steht, und
- Z für: ein Peptid mit 1 bis 5 Aminosäureresten steht, enthaltend 1 oder 2 Histidinreste.

Ferner ist ein Insulin der Formel I bevorzugt, wobei
R¹ für einen Phenylalaninrest steht, R³ für einen Glycinrest steht, Y für einen Threoninrest steht und Z für ein Peptid mit 1 bis 5 Aminosäureresten steht, enthaltend 1 oder 2 Histidinreste.

Ganz besonders bevorzugt ist ein Insulin der Formel I, wobei
Z für ein Peptid mit der Sequenz His His, His His Arg, Ala His His, Ala His His Arg, Ala Ala His His Arg oder Ala Ala His His steht.

Die Aminosäuresequenz von Peptiden und Proteinen wird vom N-terminalen Ende der Aminosäurekette an bezeichnet. Die in Formel I in Klammern gemachten Angaben, z.B. A1, A6, A7, A11, A20, B1, B7, B19 oder B30 entsprechen der Position von Aminosäureresten in den A- oder B-Ketten des Insulins.

Der Begriff "genetisch kodierbarer Aminosäurerest" steht für die Reste der Aminosäuren Gly, Ala, Ser, Thr, Val, Leu, Ile, Asp, Asn, Glu, Gln, Cys, Met, Arg, Lys, His, Tyr, Phe, Trp, Pro und Selenocystein.
Unter den Begriffen "Reste A2 - A20" und "Reste B2 - B 29" von tierischem Insulin werden beispielsweise die Aminosäuresequenzen von Insulin aus Rindern, Schweinen oder Hühnern verstanden.
Der Begriff Reste A2 - A20 und B2 - B29 von Insulinderivaten steht für die entsprechenden Aminosäuresequenzen von Humaninsulin, die durch den Austausch von Aminosäuren durch andere genetisch kodierbare Aminosäuren gebildet werden.

Die A-Kette von Humaninsulin hat folgende Sequenz (Seq Id No. 1):

Gly, Ile, Val, Glu, Gln, Cys, Cys, Thr, Ser, Ile, Cys, Ser, Leu,
Tyr, Gln, Leu, Glu, Asn, Tyr, Cys, Asn.

Die B-Kette von Humaninsulin hat folgende Sequenz (Seq ld No. 2 ):

Phe, Val, Asn, Gln, His, Leu, Cys, Gly, Ser, His, Leu, Val, Glu,
Ala, Leu, Tyr, Leu, Val, Cys, Gly, Glu, Arg, Gly, Phe, Phe, Tyr,
Thr, Pro, Lys, Thr.

Das Insulinderivat der Formel I kann mit Hilfe einer Vielzahl gentechnischer Konstrukte in Mikroorganismen gebildet werden (EP 0 489 780, EP 0 347 781, EP 0 368 187, EP 0 453 969). Die gentechnischen Konstrukte werden in Mikroorganismen wie Escherichia coli oder Streptomyceten während der Fermentation exprimiert. Die gebildeten Proteine werden im Inneren der Mikroorganismen abgelagert (EP 0 489 780) oder in die Fermentationslösung ausgeschieden.

Beispielhafte Insuline der Formel I sind:

Gly(A21)-Humaninsulin-His(B31)-His(B32)-OH
Gly(A21)-Humaninsulin-His(B31)-His(B32)-Arg (B33)-OH
Gly(A21)-Humaninsulin-Ala(B31)-His(B32)-His(B33)-OH
Gly(A21)-Humaninsulin-Ala(B31)-His(B32)-His(B33)-Arg(B34)-OH
Gly(A21)-Humaninsulin-Ala(B31)-Ala(B32)-His(B33)-His(B34)-OH
Gly(A21)-Humaninsulin-Ala(B31)-Ala(B32)-His(B33)-His(B34)-Arg(B35)-OH

Die Herstellung der Insulin-Derivate der Formel I erfolgt hauptsächlich gentechnologisch mittels site-directed mutagenesis nach Standardmethoden. Dazu wird eine für das gewünschte Insulin-Derivat der Formel I codierende Genstruktur konstruiert und in einer Wirtszelle - vorzugsweise in einem Bakterium wie E. coli oder einer Hefe, insbesondere Saccharomyces cerevisiae - zur Expression gebracht und - falls die Genstruktur für ein Fusionsprotein codiert - aus dem Fusionsprotein das Insulin-Derivat der Formel I freigesetzt; analoge Methoden sind z.B. beschrieben in EP-A-0 211 299, EP-A-0 227 938, EP-A-0 229 998, EP-A-0 286 956 und der DE-Patentanmeldung P 38 21 159.

Die Abspaltung des Fusionsproteinanteils erfolgt nach Zellenaufschluß entweder chemisch mittels Halogencyan -siehe EP-A-0 180 920 - oder enzymatisch mittels Lysostaphin oder Trypsin - siehe DE-A-37 39 347.

Der Insulinvorläufer wird dann der oxidativen Sulfitolyse nach der z.B. von R.C. Marshall und A.S. Inglis in "Practical Protein Chemistry - A Handbook" (Herausgeber A. Darbre) 1986, Seiten 49 - 53 beschriebenen Methode unterworfen und anschließend in Gegenwart eines Thiols unter Ausbildung der korrekten Disulfidbrücken renaturiert, z.B. nach der von G.H. Dixon und A.C. Wardlow in Nature (1960), Seiten 721- 724 beschriebenen Methode.

Die Insulinvorläufer können jedoch auch direkt gefaltet werden (EP-A-0 600 372 ; EP-A-0 668 292).

Das C-Peptid und - falls vorhanden - die Präsequenz (R² in Formel II) wird mittels tryptischer Spaltung entfernt - z.B. gemäß der Methode von Kemmler et al., J.B.C. (1971), Seiten 6786 - 6791 und das Insulinderivat der Formel I mittels bekannter Techniken wie Chromatographie - z.B. EP-A-0 305 760 - und Kristallisation gereinigt.

Die Erfindung betrifft ferner Komplexe, enthaltend ein Insulinhexamer und etwa 5 bis 9 Mol Zink pro Insulinhexamer, bevorzugt 5 bis 7 Mol Zink⁺⁺ pro Insulinhexamer, wobei das Insulinhexamer aus sechs Insulinmolekülen der Formel I besteht.

Die Zinkbindung an das Insulinhexamer ist so fest, daß die 5 bis 9 Mol Zink⁺⁺ pro Mol Insulinhexamer nicht durch 40 Stunden übliche Dialyse, beispielsweise mit einem wäßrigen 10 mM Tris/HCl-Puffer, pH 7,4, entfernt werden können.

Insuline der Formel zeigen nach subkutaner Applikation in einer im wesentlichen zinkfreien Zubereitung (pH 4); eine geringe Wirkungsverzögerung im Vergleich mit Humaninsulin. Nach Zusatz von etwa 20 µg Zink ²⁺/ml Zubereitung wird nach subkutaner Applikation ein späterer Wirkungseintritt beobachtet. Die Wirkungsverzögerung wird bevorzugt bei 40 µg Zink²⁺/ml beobachtet. Höhere Zinkkonzentrationen verstärken diesen Effekt.

Die Erfindung betrifft ferner Präproinsulin der Formel II,

R²-R¹-B2-B29-Y-Z¹-Gly-A2-A20-R³ (II)

wobei R³ und Y wie in Formel I gemäß einem oder mehreren der Ansprüche 1 bis 6 definiert sind, und
- R¹ für: einen Phenylalaninrest oder eine kovalente Bindung steht, und
- R² für: a) einen genetisch kodierbaren Aminosäurerest oder
b) ein Peptid mit 2 bis 45 Aminosäureresten steht, und die Reste A2 A20 und B2 - B29 den Aminosäuresequenzen der A- und B-Ketten von Humaninsulin, tierischen Insulin oder einem Insulinderivat entsprechen und wobei
- Z¹: ein Peptid mit 2 bis 40 genetisch kodierbaren Aminosäureestem mit 1 bis 5 Histidin (His)-Resten bedeutet.

Das Proinsulin der Formel II eignet sich als Zwischenverbindung bei der Herstellung der Insuline der Formel I.

Bevorzugt sind Proinsuline der Formel II, wobei
- R² für: ein Peptid mit 2 bis 25 Aminosäureresten steht.

Insbesondere bevorzugt sind Proinsuline der Formel II, wobei
- R² für: ein Peptid mit 2 bis 15 Aminosäureresten steht, worin am Carboxylende ein Aminosäurerest aus der Gruppe Met, Lys und Arg steht.

Die erfindungsgemäßen Insulin-Derivate der Formel I und/oder die Komplexe, enthaltend ein Insulinhexamer und 5 bis 9 Mol Zink⁺⁺ pro Hexamer und/oder deren physiologisch verträglichen Salze (z.B. die Alkali- oder Ammoniumsalze) werden hauptsächlich als Wirkstoffe für eine pharmazeutische Zubereitung zur Behandlung des Diabetes, insbesondere des Diabetes mellitus, verwendet.

Die pharmazeutische Zubereitung ist vorzugsweise eine Lösung oder Suspension zu Injektionszwecken; sie ist gekennzeichnet durch einen Gehalt an mindestens einem Insulin-Derivat der Formel I und/oder dem erfindungsgemäßen Komplex und/oder mindestens einem von deren physiologisch verträglichen Salzen in gelöster, amorpher und/oder kristalliner - vorzugsweise in gelöster - Form.

Die Zubereitung weist vorzugsweise einen pH-Wert von etwa 2,5 bis 8,5, insbesondere von etwa 4,0 bis 8,5, auf, enthält ein geeignetes Isotoniemittel, ein geeignetes Konservierungsmittel und gegebenenfalls einen geeigneten Puffer, sowie vorzugsweise auch eine bestimmte Zinkionen-Konzentration, alles natürlich in steriler wäßriger Lösung. Die Gesamtheit der Zubereitungsbestandteile außer dem Wirkstoff bildet die Trägerlösung der Zubereitung.

Zubereitungen, enthaltend Lösungen des Insulins der Formel I weisen einen pH-Wert von 2,5 bis 4,5 auf, insbesondere von 3,5 bis 4,5, bevorzugt 4,0. Zubereitungen, enthaltend Suspensionen des Insulins der Formel I weisen einen pH-Wert von 6,5 bis 8,5 auf, insbesondere von 7,0 bis 8,0, bevorzugt 7,4.

Geeignete Isotoniemittel sind z.B. Glycerin, Glukose, Mannit, NaCl, Calcium- oder Magnesium-Verbindungen wie CaCl₂ oder MgCl₂.

Durch die Wahl des Isotoniemittels und/oder Konservierungsstoffes beeinflußt man die Löslichkeit des Insulin-Derivats oder dessen physiologisch verträglichen Salzes bei den schwach sauren pH-Werten.

Geeignete Konservierungsmittel sind z.B. Phenol, m-Cresol, Benzylalkohol und/oder p-Hydroxybenzoesäureester.

Als Puffersubstanzen, insbesondere zur Einstellung eines pH-Wertes zwischen etwa 4,0 und 8,5, können z.B. Natriumacetat, Natriumcitrat oder Natriumphosphat verwendet werden. Ansonsten sind zur Einstellung des pH-Wertes auch physiologisch unbedenkliche verdünnte Säuren (typischerweise HCI) oder Laugen (typischerweise NaOH) geeignet.

Wenn die Zubereitung einen Zinkgehalt besitzt, ist ein solcher von 1 µg bis 2 mg Zink⁺⁺ /ml, insbesondere von 5 µg bis 200 µg Zink/ml bevorzugt.

Zwecks Variation des Wirkungsprofils der erfindungsgemäßen Zubereitung kann auch unmodifiziertes Insulin, vorzugsweise Rinder-, Schweine- oder Human-Insulin, insbesondere Human-Insulin, oder modifizierte Insuline, beispielsweise monomere Insuline, schnellwirksame Insuline oder Gly(A21)-Arg(B31)-Arg(B32)-Humaninsulin, zugemischt werden.

Bevorzugte Wirkstoffkonzentrationen sind solche entsprechend etwa 1 - 1500, weiter bevorzugt etwa 5 -1000 und insbesondere etwa 40 - 400 internationale Einheiten/ml.

### Beispiel 1

### Herstellung von Gly(A21)-Humaninsulin-His(B31)-His(B32)-OH

Die Herstellung des Expressionssystems erfolgte im wesentlichen gemäß
US 5,358,857. Dort werden auch die Vektoren pINT 90d und pINT 91d (siehe Beispiel 17) und die PCR Primer TIR und Insu11 beschrieben. Diese vier Komponenten dienen unter anderem als Ausgangsmaterial für die im folgenden beschriebenen Vektorkonstruktionen.

Zunächst wird in die das Mini-Proinsulin kodierende Sequenz das Codon für Gly (A21) eingeführt. Dazu wird pINT 91 d als Template verwendet und eine PCR-Reaktion mit den Primern TIR und Insu31 
Insu31 (Seq Id No. 10):

5'TTT TTT GTC GAC CTA TTA GCC GCA GTA GTT CTC CAG CTG 3'
 durchgeführt. Der PCR-Zyklus wird wie folgt durchgeführt. 1. Minute 94°C, 2. Minute 55°C, 3. Minute 72°C. Dieser Zyklus wird 25 mal wiederholt, dann wird 7 Minuten bei 72°C und anschließend über Nacht bei 4°C inkubiert.

Das entstandene PCR-Fragment wird zur Reinigung in Ethanol gefällt, getrocknet und anschließend in Restriktionspuffer entsprechend den Angaben der Hersteller mit den Restriktionsenzymen NcO1 und Sal1 verdaut. Das Reaktionsgemisch wird anschließend gelelektrophoretisch aufgetrennt und das NcO1-Prä-Proinsulin-SaI1 Fragment isoliert. DNA des Plasmides pINT90d wird ebenfalls mit NcO1 und SaI1 gespalten und das Affenproinsulinfragment auf diese Weise von dem pINT-Restplasmid freigesetzt. Beide Fragmente werden gelelektrophoretisch aufgetrennt und die Restplasmid-DNA isoliert. Diese DNA wird mit dem NcO1-Sal1-PCR-Fragment in einer Ligasereaktion umgesetzt. Man erhält so das Plasmid pINT150d, das nach Transformation nach E.coli dort vermehrt und anschließend reisoliert wird.

DNA des Plasmids pINT150d dient als Ausgangsmaterial für das Plasmid pINT302, das die Herstellung der gewünschten Insulinvariante erlaubt.

Zur Konstruktion dieses Plasmids wird der in US 5,358,857 (siehe Beispiel 6) beschriebene Weg eingeschlagen. Zwei voneinander unabhängige PCR-Reaktionen werden dazu durchgeführt, denen DNA des Plasmides pINT150d als Template dient. Die eine Reaktion wird mit dem Primerpaar TIR und pINT B5 
(Seq Id No. 11):

5' GAT GCC GCG GTG GTG GGT CTT GGG TGT GTAG 3'
 und die andere Reaktion mit dem Primerpaar Insu11 und pINT B6 
(Seq Id No. 12):

5' A CCC AAG ACC CAC CAC CGC GGC ATC GTG GAG 3'
 durchgeführt.

Die PCR-Fragmente, die entstehen, sind partiell komplementär, so daß sie in einer dritten PCR-Reaktion zu einem Fragment führen, das für ein um die Position B31 und B32 verlängertes Gly (A21) Miniproinsulin kodiert. Dieses Fragment wird mit NcO1 und Sal1 gespalten und anschließend mit der DNA des beschriebenen pINT90d Restplasmids in einer Ligasereaktion zu Plasmid pINT302 umgesetzt. Ein mit diesem Plasmid transformierter E.coli K12 W3110 wird anschließend wie in Beispiel 4 von US 5,227,293 fermentiert und aufgearbeitet. Das als Zwischenprodukt erhaltene Präproinsulinderivat (vor Trypsinspaltung) hat die folgende Aminosäuresequenz:

Präproinsulin 1(Seq Id No. 3):
Met Ala Thr Thr Ser Thr Gly Asn Ser Ala Arg
Phe Val Asn Gln His Leu Cys Gly Ser His Leu Val Glu Ala Leu
Tyr Leu Val Cys Gly Glu Arg Gly Phe Phe Tyr Thr Pro Lys Thr
His His Arg
Gly Ile Val Glu Gln Cys Cys Thr Ser Ile Cys Ser
Leu Tyr Gln Leu Glu Asn Tyr Cys Gly

Präproinsulin 1 entspricht der Formel II, dabei ist
- R²: ein Peptid mit 11 Aminosäureresten,
- R¹: Phe (B1),
- Y: Thr (B30),
- Z¹: His His Arg (B31-B33),
- R³: Gly (A21) und
A2-A20 ist die Aminosäuresequenz der A-Kette von Humaninsulin (Aminosäurereste 2 bis 20) und B2-B29 sind die Aminosäuresequenz der B-Kette von Humaninsulin (Aminosäurereste 2 bis 29).

Das Präproinsulin 1 wird wie in US 5, 227, 293 gemäß Beispiel 4 mit Trypsin gespalten. Das erhaltene Produkt wird anschließend mit Carboxypeptidase B gemäß Beispiel 11 zu Insulin 1 umgesetzt. Insulin 1 entspricht der Formel I, dabei ist
- R¹: Phe (B1),
- Y: Thr (B30),
- Z: His His (B31-B32),
- R³: Gly (A21) und
A2-A20 sind die Aminosäuresequenz der A-Kette von Humaninsulin (Aminosäurereste 2 bis 20) und B2-B29 sind die Aminosäuresequenz der B-Kette von Humaninsulin (Aminosäurereste 2 bis 29).

Insulin 1 hat die folgende Aminosäuresequenz: 
Insulin 1 (Seq Id No. 4):
B-Kette: Phe Val Asn Gln His Leu Cys Gly Ser His Leu Val Glu Ala Leu
 
Tyr Leu Val Cys Gly Glu Arg Gly Phe Phe Tyr Thr Pro Lys Thr
His His
A-Kette: Gly Ile Val Glu Gln Cys Cys Thr Ser Ile Cys Ser
Leu Tyr Gln Leu Glu Asn Tyr Cys Gly
 (Disulfidbrücken wie in Formel I dargestellt.)

### Beispiel 2

### Herstellung von Gly(A21 )-Humaninsulin-Ala(B31)-His(B32)-His(B33)-Arg(B34)-OH

Der Expressionsvektor wird entsprechend Beispiel 1 konstruiert.
Plasmid pINT150d dient als Template für zwei voneinander unabhängige PCR-Reaktionen mit den Primerpaaren TIR und pINT B7 (Seq Id No. 13): 5' GAT GCC GCG GTG GTG CGC GGT CTT GGG TGT GTAG 3' bzw. Insu11 und pINT B8 (Seq Ind No. 14):

5' ACCC AAG ACC GCG CAC CAC CGC GGC ATC GTG GAG 3'.

Die PCR Fragmente, zu denen beide Reaktionen führen, sind teilweise komplementär und ergeben in einer dritten PCR-Reaktion die komplette Sequenz, die für die gewünschte Insulinvariante kodiert. Das Fragment der Reaktion wird mit den Enzymen Nc01 und SaI1 behandelt und anschließend in das NcO1/SaI1 geöffnete Restplasmid der pINT90d DNA ligiert. Es entsteht Plasmid pINT303, das nach Transformation von E.coli K12 W3110 als Basis für die Expression des gewünschten Prä-Miniproinsulins dient. Die Fermentation und Aufarbeitung erfolgt wie in Beispiel 1, wobei die Carboxypeptidase B-Reaktion entfällt.
Das erhaltene Präproinsulinderivat hat die folgende Aminosäuresequenz:

Präproisulin 2 (Seq Id No. 5):
Met Ala Thr Thr Ser Thr Gly Asn Ser Ala Arg
Phe Val Asn Gln His Leu Cys Gly Ser His Leu Val Glu Ala Leu
Tyr Leu Val Cys Gly Glu Arg Gly Phe Phe Tyr Thr Pro Lys Thr

Ala His His Arg
Gly Ile Val Glu Gln Cys Cys Thr Ser Ile Cys Ser
Leu Tyr Gln Leu Glu Asn Tyr Cys Gly

Präproinsulin 2 entspricht der Formel II, dabei ist
- R¹: Phe (B1),
- R²: ein Peptid mit 11 Aminosäureresten,
- Y: Thr (B30),
- Z¹: Ala His His Arg (B31-B34),
- R³: Gly (A21) und
A2-A20 sind die Aminosäuresequenz der A-Kette von Humaninsulin (Aminosäurereste 2 bis 20) und B2-B29 sind die Aminosäuresequenz der B-Kette von Humaninsulin (Aminosäurereste 2 bis 29).

Das Präproinsulin 2 wird anschließend mit Trypsin zu Insulin 2 umgesetzt. Insulin 2 entspricht der Formel II, dabei ist
- R¹: Phe (B1),
- Y: Thr (B30),
- Z¹: Ala His His Arg (B31-B34),
- R³: Gly (A21 ) und
A2-A20 sind die Aminosäuresequenz der A-Kette von Humaninsulin (Aminosäurereste 2 bis 20) und B2-B29 sind die Aminosäuresequenz der B-Kette von Humaninsulin (Aminosäurereste 2 bis 29).

Insulin 2 hat die folgende Aminosäuresequenz: 
Insulin 2 (Seq Ind No. 6):
B-Kette: Phe Val Asn Gln His Leu Cys Gly Ser His Leu Val Glu Ala Leu
Tyr Leu Val Cys Gly Glu Arg Gly Phe Phe Tyr Thr Pro Lys Thr
Ala His His Arg
A-Kette: Gly Ile Val Glu Gln Cys Cys Thr Ser Ile Cys Ser
Leu Tyr Gln Leu Glu Asn Tyr Cys Gly
 (Disulfidbrücken wie in Formel I dargestellt.)

### Beispiel 3

### Herstellung von Gly(A21)-Humaninsulin-Ala(B31)-Ala (B32)-His(B33)-His(B34)-OH

Der Expressionsvektor wird entsprechend Beispiel 1 konstruiert. Plasmid pINT150d dient als Template für zwei voneinander unabhängige PCR-Reaktionen mit den Primerpaaren TIR und pINT 316a (Seq Ind No. 15): 5' GAT GCC GCG ATG ATG CGC CGC GGT CTT GGG TGT GTA G 3' bzw. Insu11 und pINT 316b (Seq Ind No. 16):

5' A CCC AAG ACC GCG GCG CAT CAT CGC GGC ATC GTG GAG 3'.

Die PCR Fragmente, zu denen beide Reaktionen führen, sind teilweise komplementär und ergeben in einer dritten PCR-Reaktion die komplette Sequenz, die für die gewünschte Insulinvariante kodiert. Das Fragment der Reaktion wird mit den Enzymen Nc01 und SaI1 behandelt und anschließend in das NcO1/SaI1 geöffnete Restplasmid der pINT90d DNA ligiert. Es entsteht Plasmid pINT316, das nach Transformation von E.coli K12 W3110 als Basis für die Expression des gewünschten Prä-Miniproinsulins dient. Die Fermentation und Aufarbeitung erfolgt wie in Beispel 1, wobei die Carboxypeptidase B-Reaktion entfällt.
Das erhaltene Präproinsulin 3 hat die folgende Aminosäuresequenz:

Präproinsulin 3 (Seq Id No.7):
Met Ala Thr Thr Ser Thr Gly Asn Ser Ala Arg
Phe Val Asn Gln His Leu Cys Gly Ser His Leu Val Glu Ala Leu
Tyr Leu Val Cys Gly Glu Arg Gly Phe Phe Tyr Thr Pro Lys Thr
Ala Ala His His Arg
Gly Ile Val Glu Gln Cys Cys Thr Ser Ile Cys Ser
Leu Tyr Gln Leu Glu Asn Tyr Cys Gly

Präproinsulin 3 entspricht der Formel II, dabei ist
- R¹: Phe (B1),
- R²: ein Peptid mit 11 Aminosäureresten,
- Y: Thr (B30),
- Z¹: Ala Ala His His Arg (B31-B35),
- R³: Gly (A21) und
A2-A20 sind die Aminosäuresequenz der A-Kette von Humaninsulin (Aminosäurereste 2 bis 20) und B2-B29 sind die Aminosäuresequenz der B-Kette von Humaninsulin (Aminosäurereste 2 bis 29).

Das Präproinsulin 3 wird anschließend mit Trypsin und Carboxypeptidase B wie in Beispiel 11 zu Insulin 3 umgesetzt.
Insulin 3 entspricht der Formel I, dabei ist
- R¹: Phe (B1),
- Y: Thr (B30),
- Z: Ala Ala His His (B31-B34),
- R³: Gly (A21) und
A2-A20 sind die Aminosäuresequenz der A-Kette von Humaninsulin (Aminosäurereste 2 bis 20) und B2-B29 sind die Aminosäuresequenz der B-Kette von Humaninsulin (Aminosäurereste 2 bis 29).

Insulin 3 hat die folgende Aminosäuresequenz: 
Insulin 3 (Seq Ind No. 8):
B-Kette: Phe Val Asn Gln His Leu Cys Gly Ser His Leu Val Glu Ala Leu
Tyr Leu Val Cys Gly Glu Arg Gly Phe Phe Tyr Thr Pro Lys Thr
Ala Ala His His
A-Kette: Gly Ile Val Glu Gln Cys Cys Thr Ser Ile Cys Ser
Leu Tyr Gln Leu Glu Asn Tyr Cys Gly
 (Disulfidbrücken wie in Formel I dargestellt.)

### Beispiel 4

Insulin 2 hergestellt gemäß Beispiel 2 wird mit Carboxypeptidase B gemäß Beispiel 11 zu Insulin 4 umgesetzt.
Insulin 4 entspricht der Formel I, dabei ist
- R¹: Phe (B1),
- Y: Thr (B30),
- Z: Ala His His (B31-B33),
- R³: Gly (A21) und
A2-A20 sind die Aminosäuresequenz der A-Kette von Humaninsulin (Aminosäurereste 2 bis 20) und B2-B29 sind die Aminosäuresequenz der B-Kette von Humaninsulin (Aminosäurereste 2 bis 29).

Insulin 4 hat die folgende Aminosäuresequenz: 
Insulin 4 (Seq Ind No. 9):
B-Kette: Phe Val Asn Gln His Leu Cys Gly Ser His Leu Val Glu Ala Leu
Tyr Leu Val Cys Gly Glu Arg Gly Phe Phe Tyr Thr Pro Lys Thr
Ala His His
A-Kette: Gly Ile Val Glu Gln Cys Cys Thr Ser Ile Cys Ser
Leu Tyr Gln Leu Glu Asn Tyr Cys Gly
 (Disulfidbrücken wie in Formel I dargestellt.)

### Beispiel 5

### Zinkbindung von Insulinderivaten

Eine Zubereitung von Insulin (0,243 mM Human-Insulin, 0,13 M NaCl, 0,1 % Phenol, 80 µg/ml (1,22 mM) Zink⁺⁺, 10 mM Tris/HCl, pH 7,4) wird bei 15°C gegen 10 mM Tris/HCl pH 7,4 insgesamt 40 Stunden (Pufferwechsel nach 16 h und 24 h) dialysiert. Danach werden die Dialysate angesäuert, die Konzen-tration von Insulin durch HPLC und Zink durch Atomabsorptions-spektroskopie bestimmt. Die Zinkwerte werden mit dem Zinkgehalt eines Kontrollansatzes, der kein Insulin enthält, korrigiert. Tabelle 1 zeigt die Ergebnisse:

**Tabelle 1:**

| Vergleichsinsuline | mol Zn/ mol Insulinhexamer |
|---|---|
| Humaninsulin (HI) | 1,98 |
| Gly(A21)Des(B30)-HI | 1,8 |
| Gly(A21)Arg(B31)-Arg(B32)-HI | 2,1 |

Erfindungsgemäße Insuline der Formel I:

| Insulin der Formel I | mol Zn/ mol Insulinhexamer |
|---|---|
| Gly(A21)His(B31)His(B32)-HI | 6,53 |
| Gly(A21)His(B31)His(B32)Arg(B33)-HI | 5,29 |
| Gly(A21)Ala(B31 )His(B32)His(B33)-HI | 6,73 |
| Gly(A21)Ala(B31)His(B32)His(B33)Arg(B34)-HI | 5,01 |

### Beispiel 6

Zinkabhängigkeit des Wirkprofils von Human-Insulin am Hund

| | |
|---|---|
| Applikation | subkutan |
| Dosis | 0,3 IE/kg; pH der Zubereitung 4,0 |

Anzahl der Hunde (n) pro Versuch ist 6

Tabelle 2 zeigt die Blutglukose in % des Ausgangswertes.

**Tabelle 2**

| Zeit (h) | zinkfrei | 80 µg Zn/ml | 160 µg Zn/ml |
|---|---|---|---|
| 0 | 100 | 100 | 100 |
| 0,5 | 88,66 | 94,06 | 101,48 |
| 1 | 59,73 | 72,31 | 76,87 |
| 1,5 | **50,61** | 58,6 | 67,44 |
| 2 | 54,32 | **54,05** | **61,49** |
| 3 | 61,94 | 58,84 | 62,8 |
| 4 | 85,59 | 70,03 | 71,32 |
| 5 | 100,46 | 78,97 | 81,65 |
| 6 | 105,33 | 94,63 | 96,19 |
| 7 | 106 | 102,46 | 100,27 |
| 8 | 108,39 | 106,12 | 104,34 |
| 10 | 102,72 | 105,11 | 105,1 |
| 12 | 105,03 | 107,14 | 103,02 |

### Beispiel 7

### Wirkprofil von Gly(A21)Ala(B31 )His(B32),His(B33),Arg(B34)-Human-Insulin am Hund (Insulin 2)

Das Insulin 2 hergestellt gemäß Beispiel 2 wird in folgender Formulierung eingesetzt:
Glycerin 20 mg/ml, m-Kresol 2,7 mg/ml, Insulin 2 40 IE/ml
   IE steht für Internationale Einheiten und entspricht etwa 6 nmol Insulin, z.B. Humaninsulin oder Insulin der Formel I. Der pH wird mit NaOH oder HCl eingestellt.
Applikation: subkutan; Dosis: 0,3 IE/kg;
   die Anzahl der getesteten Hunde ist 6; pH der Zubereitung 4,0

Tabelle 3 zeigt die Blutglukose in % des Ausgangswertes.

**Tabelle 3**

| Zeit (h) | zinkfrei | 20 µg Zn/ml | 40 µg Zn/ml | 80 µg Zn/ml |
|---|---|---|---|---|
| 0 | 100 | 100 | 100 | 100 |
| 1 | 74,38 | 95,24 | 95,6 | 102,06 |
| 2 | **48,27** | 90,11 | 78,74 | 97,44 |
| 3 | 57,67 | 89,96 | 84,81 | 90,44 |
| 4 | 74,2 | 81,35 | 74,66 | 88,69 |
| 5 | 91,68 | 74,43 | 75,71 | 79,7 |
| 6 | 100,79 | 71,61 | 67,37 | 65,26 |
| 7 | 98,5 | **67,73** | 66,05 | 62,17 |
| 8 | 100,54 | 68,92 | **64,97** | **47,71** |

### Beispiel 8

### Wirkprofil von Gly(A21)Ala(B31)His(B32),His(B33)-Human-Insulin am Hund (Insulin 4)

Das Insulin 4 hergestellt gemäß Beispiel 4 wird wie in Beispiel 7 formuliert und eingesetzt.
Applikation: subkutan; Dosis: 0,3 IE/kg;
n = 6; pH der Zubereitung 4,0
Tabelle 4 zeigt die Blutglukose in % des Ausgangswertes.

**Tabelle 4**

| Zeit (h) | zinkfrei | 20 µg Zn/ml | 40 µg Zn/ml | 80 µg Zn/ml |
|---|---|---|---|---|
| 0 | 100 | 100 | 100 | 100 |
| 1 | 61,51 | 70 | 96,52 | 101,77 |
| 2 | **49,82** | **52,55** | 89,28 | 90,01 |
| 3 | 55,66 | 60,13 | 80,23 | 70,79 |
| 4 | 78,09 | 78,46 | 73,03 | **68,48** |
| 5 | 94,27 | 97,7 | 70,3 | 74,94 |
| 6 | 103,69 | 105,27 | **61,86** | 74,1 |
| 7 | 105,51 | 106,48 | 62,28 | 76,42 |
| 8 | 108,05 | 104,51 | 81,68 | 88 |

### Beispiel 9

### Wirkprofil von Gly(A21)His(B31),His(B32)-Human-Insulin am Hund (Insulin 1)

Das Insulin 1 hergestellt gemäß Beispiel 1 wird wie in Beispiel 7 formuliert und eingesetzt.
Applikation: subkutan; Dosis: 0,3 IE/kg;
n = 6; pH der Zubereitung 4,0
Tabelle 5 zeigt die Blutglukose in % des Ausgangswertes.

**Tabelle 5**

| Zeit (h) | zinkfrei | 20 µg Zn/ml | 40 µg Zn/ml | 80 µg Zn/ml |
|---|---|---|---|---|
| 0 | 100 | 100 | 100 | 100 |
| 1 | 60,71 | 73,16 | 100,25 | 102,86 |
| 2 | **52,29** | **55,43** | 94,86 | 100,19 |
| 3 | 61,74 | 61,6 | 89,37 | 89,12 |
| 4 | 79,93 | 81,53 | 81,55 | 79,19 |
| 5 | 96,17 | 96,84 | **73,06** | **70,67** |
| 6 | 103,2 | 102,43 | 74,58 | 75,75 |
| 7 | 110,86 | 104,75 | 77,68 | 79,36 |
| 8 | 113,42 | 108,14 | 84,87 | 78,74 |

### Beispiel 10

### Wirkprofil von Gly(A21)Ala(B31)Ala(B32)His(B33)His(B34)-Human-Insulin am Hund (Insulin 3)

Das Insulin 3 hergestellt gemäß Beispiel 1 wird wie in Beispiel 7 formuliert und eingesetzt.
Applikation: subkutan; Dosis: 0,3 IE/kg;
n = 6; pH der Zubereitung 4,0
Tabelle 6 zeigt die Blutglukose in % des Ausgangswertes.

**Tabelle 6**

| Zeit (h) | zinkfrei | 20 µg Zn/ml | 40 µg Zn/ml | 80 µg Zn/ml |
|---|---|---|---|---|
| 0 | 100 | 100 | 100 | 100 |
| 1 | 75 | 99 | 101 | 99 |
| 2 | **57** | 77 | 96 | 91 |
| 3 | 58 | **64** | 84 | 80 |
| 4 | 82 | 65 | 73 | 79 |
| 5 | 94 | 70 | **68** | 77 |
| 6 | 100 | 74 | 72 | 76 |
| 7 | 96 | 81 | 80 | **69** |
| 8 | 100 | 90 | 88 | 75 |
| 9 | 100 | 96 | 94 | 83 |
| 10 | 95 | 98 | 92 | 87 |
| 12 | 98 | 99 | 95 | 94 |
| 14 | 95 | 100 | 94 | 93 |

### Beispiel 11

### Herstellung von Insulin 1 aus Präproinsulin 1

200 mg des Insulins mit Arg-am Carboxy-Terminus der B-Kette, hergestellt gemäß Beispiel 1 wird in 95 ml 10 mM HCl gelöst. Nach Zugabe von 5 ml 1 M Tris/HCl (Tris(hydroxymethyl)aminomethan) pH 8 stellt man den pH-Wert mit HCl oder NaOH auf 8.
Es werden 0,1 mg Carboxypeptidase B zugesetzt Nach 90 Minuten ist die Abspaltung des Arginins vollständig. Der Ansatz wird durch Zugabe von HCl auf pH 3,5 gestellt und auf eine Reversed Phase Säule (PLRP-S RP 300 10 u, 2,5 x 30 cm, Polymer Laboratories Amherst, MA, USA) gepumpt. Die mobile Phase A ist Wasser mit 0,1 % Trifluoressigsäure. Phase B besteht aus Acetonitril mit 0,09 % Trifluoressigsäure. Die Säule wird mit einem Fluß von 5 ml/min betrieben. Nach dem Auftragen wird die Säule mit 150 ml A gewaschen. Die fraktionierte Elution erfolgt durch Anlegen eines linearen Gradienten von 22,5 bis 40 %B in 400 Minuten. Die Fraktionen werden einzeln durch analytische Reversed Phase HPLC analysiert und diejenigen, die Des-Arg-Insulin von hinreichender Reinheit enthalten, vereinigt. Der pH-Wert wird mit NaOH auf 3,5 gestellt und das Acetonitril im Rotationsverdampfer entfernt. Anschließend wird das Des-Arg-Insulin durch Einstellen von pH 6,5 ausgefällt. Das Präzipitat wird abzentrifugiert, zweimal mit jeweils 50 ml Wasser gewaschen und zuletzt gefriergetrocknet. Die Ausbeute beträgt 60 bis 80 % von Insulin 1.

## Patentansprüche

1. Insulin der Formel I 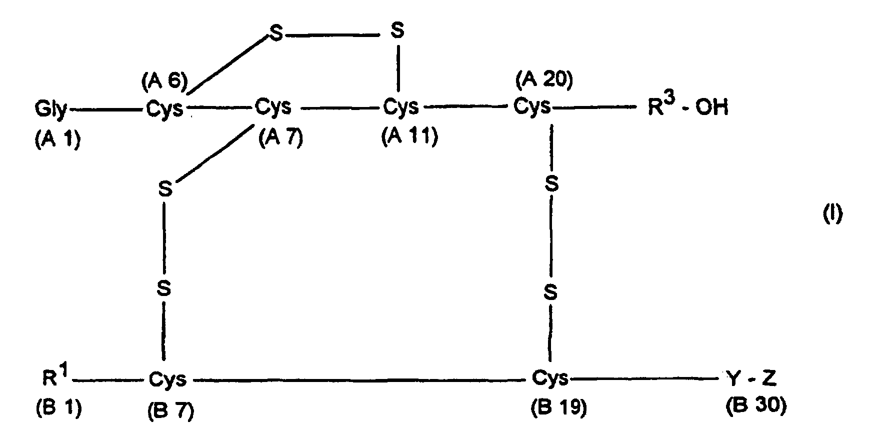 oder ein physiologisch verträgliches Salz des Insulins der Formel I, wobei
R¹ für einen Phenylalaninrest oder ein Wasserstoffatom steht,
R³ für einen genetisch kodierbaren Aminosäurerest steht,
Y für einen genetisch kodierbaren Aminosäurerest steht (Position B30),
Z für
a) einen Histidinrest oder
b) ein Peptid mit 2 bis 35 genetisch kodierbaren Aminosäureresten, enthaltend 1 bis 5 Histidinreste, steht, und
die Reste A2-A20 entsprechen der Aminosäuresequenz der A-Kette von Humaninsulin, tierischen Insulin oder einem Insulinderivat und die Reste B2-B29 entsprechen der Aminosäuresequenz der B-Kette von Humaninsulin, tierischen Insulin oder einem Insulinderivat.

2. Insulin der Formel I gemäß Anspruch 1, wobei
R¹ für Phenylalaninrest steht,
R³ für einen Aminosäurerest aus der Gruppe Asn, Gly, Ser, Thr, Ala, Asp, Glu und Gln steht,
Y für einen Aminosäurerest aus der Gruppe Ala, Thr, Ser und His steht,
Z für
a) den Histidinrest oder
b) ein Peptid mit 4 bis 7 Aminosäureresten steht, enthaltend 1 oder 2 Histidinreste.

3. Insulin der Formel I gemäß Anspruch 1, wobei
R¹ für Phenylalaninrest steht,
R³ für einen Aminosäurerest aus der Gruppe Asn, Gly, Ser, Thr, Ala, Asp, Glu und Gln steht,
Y für einen Aminosäurerest aus der Gruppe Ala, Thr, Ser und His steht,
Z für
a) den Histidinrest oder
b) ein Peptid mit 2 bis 7 Aminosäureresten steht, enthaltend 1 oder 2 Histidinreste.

4. Insulin der Formel I gemäß Anspruch 3, wobei
Z für ein Peptid mit 1 bis 5 Aminosäureresten steht, enthaltend 1 oder 2 Histidinreste.

5. Insulin der Formel I gemäß einem oder mehreren der Ansprüche 1 bis 4, wobei
R¹ für Phenylalaninrest steht,
R³ für einen Aminosäurerest aus der Gruppe Asn und Gly steht,
Y für einen Aminosäurerest aus der Gruppe Thr und His steht, und
Z für ein Peptid mit 1 bis 5 Aminosäureresten steht, enthaltend 1 oder 2 Histidinreste.

6. Insulin der Formel I gemäß Anspruch 1, wobei
R¹ für Phenylalaninrest steht, R³ für Glycinrest steht, Y für Threoninrest steht und Z für ein Peptid mit 1 bis 5 Aminosäureresten steht.

7. Insulin der Formel I gemäß Anspruch 6, wobei
Z für ein Peptid mit der Sequenz His His, His His Arg, Ala His His, Ala His His Arg, Ala Ala His His Arg oder Ala Ala His His steht.

8. Komplexe, enthaltend ein Insulinhexamer und 5 bis 9 Mol Zink pro Insulinhexamer, insbesondere 5 bis 7 Mol Zink pro Insulinhexamer, wobei das Insulinhexamer aus sechs Insulinmolekülen der Formel I gemäß einem oder mehreren der Ansprüche 1 bis 7 besteht.

9. Pharmazeutische Zubereitung, **gekennzeichnet durch** eine wirksame Menge an mindestens einem Insulin der Formel I und/oder mindestens einem physiologisch verträglichen Salz des Insulins der Formel I gemäß einem oder mehreren der Ansprüche 1 bis 8 in gelöster, amorpher und/oder kristalliner Form.

10. Pharmazeutische Zubereitung nach Anspruch 9, **gekennzeichnet durch** einen zusätzlichen Gehalt von 1 µg bis 2 mg, vorzugsweise von 5 µg bis 200 µg Zink/ml.

11. Pharmazeutische Zubereitung nach Anspruch 9 oder 10, **gekennzeichnet durch** einen pH von 2,5 bis 8,5.

12. Pharmazeutische Zubereitung nach einem oder mehreren der Ansprüche 9 bis 11, **gekennzeichnet durch** einen pH von 2,5 bis 4,5.

13. Pharmazeutische Zubereitung nach einem oder mehreren der Ansprüche 9 bis 12, **gekennzeichnet durch** einen zusätzlichen Gehalt an unmodifiziertem Insulin, vorzugsweise unmodifiziertem Humaninsulin oder modifiziertem Insulin, vorzugsweise Gly(A21)-Arg(B31)-Arg(B32)-Humaninsulin.

14. Proinsulin der Formel II,
R²-R¹-B2-B29-Y-Z¹-Gly-A2-A20-R³ (II)
wobei R³ und Y wie in Formel I gemäß einem oder mehreren der Ansprüche 1 bis 7 definiert ist,
R¹ für einen Phenylalaninrest oder eine kovalente Bindung steht,
R² für
a) einen genetisch kodierbare Aminosäurerest oder
b) ein Peptid mit 2 bis 45 Aminosäureresten steht, und die Reste A2 - A20 und B2 - B29 den Aminosäuresequenzen der A- und B-Ketten von Humaninsulin, tierischen Insulin oder einem Insulinderivat entsprechen, und
Z¹ ein Peptid mit 2 bis 40 genetisch kodierbaren Aminosäureresten mit 1 bis 5 Histidinresten bedeutet.

15. Proinsulin der Formel II gemäß Anspruch 14, wobei
R² für ein Peptid mit 2 bis 25 Aminosäureresten steht.

16. Proinsulin der Formel II gemäß Anspruch 14 oder 15, wobei
R² für ein Peptid mit 2 bis 15 Aminosäureresten steht, worin am Carboxylende ein Aminosäurerest aus der Gruppe Met, Lys und Arg steht.

## Claims

1. An insulin of the formula I 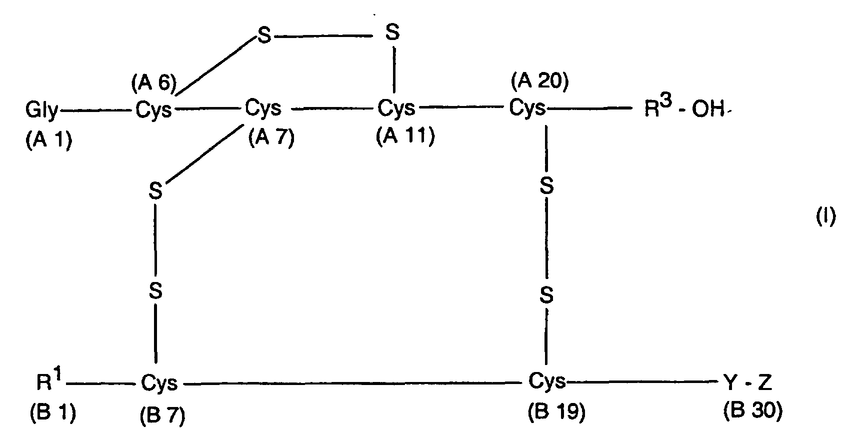 or a physiologically tolerable salt of the insulin of the formula I, where
R¹ is a phenylalanine residue or a hydrogen atom,
R³ is a genetically encodable amino acid residue,
Y is a genetically encodable amino acid residue (position B30),
Z is
a) a histidine residue or
b) a peptide having 2 to 35 genetically encodable amino acid residues, containing 1 to 5 histidine residues,
and the residues A2-A20 correspond to the amino acid sequence of the A chain of human insulin, animal insulin or an insulin derivative and the residues B2-B29 correspond to the amino acid sequence of the B chain of human insulin, animal insulin or an insulin derivative.

2. An insulin of the formula I as claimed in claim 1, where
R¹ is a phenylalanine residue,
R³ is an amino acid residue from the group consisting of Asn, Gly, Ser, Thr, Ala, Asp, Glu and Gln,
Y is an amino acid residue from the group consisting of Ala, Thr, Ser and His,
Z is
a) the histidine residue or
b) a peptide having 4 to 7 amino acid residues, containing 1 or 2 histidine residues.

3. An insulin of the formula I as claimed in claim 1, where
R¹ is a phenylalanine residue,
R³ is an amino acid residue from the group consisting of Asn, Gly, Ser, Thr, Ala, Asp, Glu and Gln,
Y is an amino acid residue from the group consisting of Ala, Thr, Ser and His,
Z is
a) the histidine residue or
b) a peptide having 2 to 7 amino acid residues, containing 1 or 2 histidine residues.

4. An insulin of the formula I as claimed in claim 3, where
Z is a peptide having 1 to 5 amino acid residues, containing 1 or 2 histidine residues.

5. An insulin of the formula I as claimed in one or more of claims 1 to 4, where
R¹ is a phenylalanine residue,
R³ is an amino acid residue from the group consisting of Asn and Gly,
Y is an amino acid residue from the group consisting of Thr and His, and
Z is a peptide having 1 to 5 amino acid residues, containing 1 or 2 histidine residues.

6. An insulin of the formula I as claimed in claim 1, where
R¹ is a phenylalanine residue, R³ is a glycine residue, Y is a threonine residue and Z is a peptide having 1 to 5 amino acid residues.

7. An insulin of the formula I as claimed in claim 6, where
Z is a peptide having the sequence His His, His His Arg, Ala His His, Ala His His Arg, Ala Ala His His Arg or Ala Ala His His.

8. A complex containing an insulin hexamer and 5 to 9 mol of zinc per insulin hexamer, in particular 5 to 7 mol of zinc per insulin hexamer, the insulin hexamer consisting of six insulin molecules of the formula I as claimed in one or more of claims 1 to 7.

9. A pharmaceutical preparation, which contains an efficacious amount of at least one insulin of the formula I and/or at least one physiologically tolerable salt of the insulin of the formula I as claimed in one or more of claims 1 to 8 in dissolved, amorphous and/or crystalline form.

10. A pharmaceutical preparation as claimed in claim 9, which has an additional content of 1 ·g to 2 mg, preferably from 5 ·g to 200 ·g, of zinc/ml.

11. A pharmaceutical preparation as claimed in claim 9 or 10, which has a pH of 2.5 to 8.5.

12. A pharmaceutical preparation as claimed in one or more of claims 9 to 11, which has a pH of 2.5 to 4.5.

13. A pharmaceutical preparation as claimed in one or more of claims 9 to 12, which has an additional content of unmodified insulin, preferably unmodified human insulin, or modified insulin, preferably Gly(A21)-Arg(B31)-Arg(B32)-human insulin.

14. A proinsulin of the formula II
R²-R¹-B2-B29-Y-Z¹-Gly-A2-A20-R³ (II)
where R³ and Y as in formula I are defined as set forth in one or more of claims 1 to 7,
R¹ is a phenylalanine residue or a covalent bond,
R² is
a) a genetically encodable amino acid residue or
b) a peptide having 2 to 45 amino acid residues, and the residues A2 - A20 and B2 - B29 correspond to the amino acid sequences of the A and B chains of human insulin, animal insulin or an insulin derivative, and
Z¹ is a peptide having 2 to 40 genetically encodable amino acids residues, having 1 to 5 histidine residues.

15. A proinsulin of the formula II as claimed in claim 14, where
R² is a peptide having 2 to 25 amino acid residues.

16. A proinsulin of the formula II as claimed in claim 14 or 15, where
R² is a peptide having 2 to 15 amino acid residues, in which an amino acid residue from the group consisting of Met, Lys and Arg is at the carboxyl end.

## Revendications

1. Insuline de formule 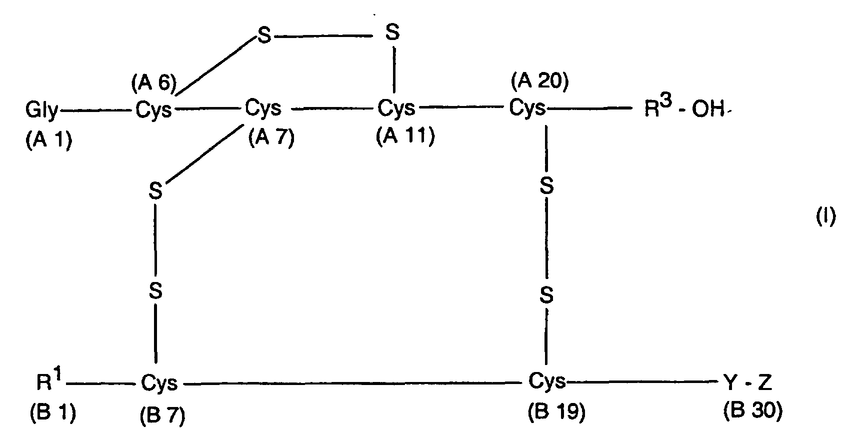 ou un sel physiologiquement acceptable de l'insuline de formule I, dans laquelle
R¹ représente un résidu de phénylalanine ou un atome d'hydrogène,
R³ représente un résidu d'aminoacide génétiquement codable,
Y représente un résidu d'aminoacide génétiquement codable (position B30),
Z représente
a) un résidu d'histidine ou
b) un peptide de 2 à 35 résidus d'aminoacides génétiquement codables, contenant 1 à 5 résidus d'histidine, et
les résidus A2-A20 correspondent à la séquence d'aminoacides de la chaîne A de l'insuline humaine, d'une insuline animale ou d'un dérivé d'insuline, et les résidus B2-B29 correspondent à la séquence d'aminoacides de la chaîne B de l'insuline humaine, d'une insuline animale ou d'un dérivé d'insuline.

2. Insuline de formule I selon la revendication 1, dans laquelle
R¹ représente un résidu de phénylalanine,
R³ représente un résidu d'aminoacide du groupe constitué par Asn, Gly, Ser, Thr, Ala, Asp, Glu et Gln,
Y représente un résidu d'aminoacide du groupe constitué par Ala, Thr, Ser et His,
Z représente
a) un résidu d'histidine ou
b) un peptide de 4 à 7 résidus d'aminoacides contenant 1 ou 2 résidus d'histidine.

3. Insuline de formule I selon la revendication 1, dans laquelle
R¹ représente un résidu de phénylalanine,
R³ représente un résidu d'aminoacide du groupe constitué par Asn, Gly, Ser, Thr, Ala, Asp, Glu et Gln,
Y représente un résidu d'aminoacide du groupe constitué par Ala, Thr, Ser et His,
Z représente
a) un résidu d'histidine ou
b) un peptide de 2 à 7 résidus d'aminoacides contenant 1 ou 2 résidus d'histidine.

4. Insuline de formule I selon la revendication 3, dans laquelle
Z représente un peptide de 1 à 5 résidus d'aminoacides contenant 1 ou 2 résidus d'histidine.

5. Insuline de formule I selon l'une ou plusieurs des revendications 1 à 4, dans laquelle
R¹ représente un résidu de phénylalanine,
R³ représente un résidu d'aminoacide du groupe constitué par Asn et Gly,
Y représente un résidu d'aminoacide du groupe constitué par Thr et His, et
Z représente un peptide de 1 à 5 résidus d'aminoacides contenant 1 ou 2 résidus d'histidine.

6. Insuline de formule I selon la revendication 1, dans laquelle R¹ représente un résidu de phénylalanine, R³ représente un résidu de glycine, Y représente un résidu de thréonine et Z représente un peptide de 1 à 5 résidus d'aminoacides.

7. Insuline de formule I selon la revendication 6, dans laquelle Z représente un peptide ayant la séquence His-His, His-His-Arg, Ala-His-His, Ala-His-His-Arg, Ala-Ala-His-His-Arg ou Ala-Ala-His-His.

8. Complexes contenant un hexamère d'insuline et 5 à 9 mol de zinc par hexamère d'insuline, en particulier 5 à 7 mol de zinc par hexamère d'insuline, l'hexamère d'insuline étant constitué de 6 molécules d'insuline de formule I selon l'une ou plusieurs des revendications 1 à 7.

9. Composition pharmaceutique **caractérisée par** une quantité efficace d'au moins une insuline de formule I et/ou d'au moins un sel physiologiquement acceptable de l'insuline de formule I selon l'une ou plusieurs des revendications 1 à 8 sous forme dissoute, amorphe et/ou cristalline.

10. Composition pharmaceutique selon la revendication 9, **caractérisée en ce qu'**elle contient en outre 1 µg à 2 mg, de préférence 5 µg à 200 µg, de zinc/ml.

11. Composition pharmaceutique selon la revendication 9 ou 10, **caractérisée par** un pH de 2,5 à 8,5.

12. Composition pharmaceutique selon l'une ou plusieurs des revendications 9 à 11, **caractérisée par** un pH de 2,5 à 4,5.

13. Composition pharmaceutique selon l'une ou plusieurs des revendications 9 à 12, **caractérisée en ce qu'**elle contient en outre une insuline non modifiée, de préférence de l'insuline humaine non modifiée, ou une insuline modifiée, de préférence la Gly(A21)-Arg(B31)-(Arg(B32)-insuline humaine.

14. Proinsuline de formule II
R²-R¹-B2-B29-Y-Z¹-Gly-A2-A20-R³ (II)
dans laquelle R³ et Y ont la définition donnée pour la formule I selon une ou plusieurs des revendications 1 à 7,
R¹ représente un résidu de phénylalanine ou une liaison covalente,
R² représente
a) un résidu d'aminoacide génétiquement codable, ou
b) un peptide de 2 à 45 résidus d'aminoacides, et
les résidus A2-A20 et B2-B29 correspondent aux séquences d'aminoacides des chaînes A et B de l'insuline humaine, d'une insuline animale ou d'un dérivé d'insuline, et
Z¹ représente un peptide de 2 à 40 résidus d'aminoacides génétiquement codables, contenant 1 à 5 restes d'histidine.

15. Proinsuline de formule II selon la revendication 14, dans laquelle R² représente un peptide de 2 à 25 résidus d'aminoacides.

16. Proinsuline de formule II selon la revendication 14 ou 15, dans laquelle R² représente un peptide de 2 à 15 résidus d'aminoacides, dans lequel un résidu d'aminoacide du groupe constitué par Met, Lys et Arg se trouve à l'extrémité carboxyle.
